# EUROPEAN PATENT APPLICATION

(11) **EP 1 356 808 A2**
(43) Date of publication of application: **29.10.2003**
(21) Application number: 03252647.7
(22) Date of filing: 25.04.2003
(51) Int. Cl.: A61K 9/28, A61K 9/24

(54) **Coating technique for deposition of drug substance on a substrate**

(30) Priority: 26.04.2002 US 134305
(71) Applicant: ETHICON, INC., Somerville, NJ 08876 (US)
(72) Inventor: Verreck, Geert, 2390 Oostmalle (BE); Rosenblatt, Joel, Watchung, New Jersey 07060 (US); Liland, Alfred, Newton, New Jersey 07860 (US)
(74) Representative: Mercer, Christopher Paul

(57) **Abstract**

The present invention relates to a multi-layered, physiologically tolerated oral dosage form for pharmaceutically active compounds. The dosage form comprises a central core, a middle layer, and an outer shell, at least one of which includes at least one pharmaceutically active substance. By varying the diameter of the core, a different middle layer volume is obtained within a fixed outer shell dimension. This gives the ability to obtain different dosage strengths for one composition without the need of reformulation work. The oral dosage form is produced in a single-step, continuous process by coating the core with the middle layer and the outer shell.

## Description

### FIELD OF THE INVENTION

The present invention relates to a multi-layered, physiologically tolerated oral dosage tablet for pharmaceutically active compounds and a method of making the tablet.

### BACKGROUND OF THE INVENTION

Classical tablet production involves a number of steps carried out in a batch-wise manner. Traditionally, the tableting process consists of different blending steps eventually combined with a wet granulation step, a tablet compression step and a film coating step. Recently, melt extrusion has been introduced in the pharmaceutical industry to combine these steps in one simple, continuous process to produce tablets.

For instance U.S. patents 4,880,585 and 5,073,379 (to Klimesch et al.) both describe a continuous process using a melt extruder to blend and melt a pharmaceutically active compound with one or more thermoplastic polymers and whereby tablets are formed on-line between two belts, a belt and a roller, or two rollers which are drawn in opposite directions.

U.S. patent 6,051,253 (to Zetter et al.) describes a continuous process using a melt extruder to blend and melt a pharmaceutically active compound with one or more thermoplastic polymers. Tablets are formed on-line in two steps, with the extrudate being broken into shaped articles in a first step, and these shaped articles being rounded off in a second step.

WO 99/02136 (to Engle et al.) relates to a multi-layered presentation form for medicines which is produced using a method whereby a core component and a coating component are injected into a shared tool cavity in such a way that the core component is fully coated by the coating component. In this process, two extruders are used to inject the two melt streams in the shared tool cavity.

WO 97/15293 (to Breitenbach et al.) describes a method to produce multi-layer medicaments whereby at least two thermoplastic, polymeric substances of which at least one contains a pharmaceutically active substance are co-extruded and the co-extruded multi-layer material is shaped to form the desired medicament. The different layers of the medicament provide for targeting the desired release: i.e., thickness of layer and polymer selection will define the release of the pharmaceutically active substance.

WO 98/27927 (to O'Donoghue et al.) provides a method for coating a core material and compressing and sealing the coating structure further downstream. The coating process is preferably done using an extruder, whereby the core material is introduced through a nozzle at the end of the extruder. The core material can take a number of physical forms, such as a tablet, a gel, a paste, or a powder. The purpose of the coating is to provide for control or delay of the release of a pharmacologically active material.

The art discussed above describes continuous processes for the production of pharmaceutical dosage forms compared to the classical batch-wise tablet production. It is often required to produce dosage forms of a pharmaceutically active substance with different doses. This may be necessary for clinical trials to test the efficacy of different doses as well as for commercial dosage forms depending on the application. Moreover, in a number of cases these dosage forms need to have the same dimensions for the different dosage strengths. For instance, in double blind clinical studies it is necessary to provide tablets with the same dimensions and nominal weight for the whole set of doses to be tested in the study.

Changing the dosage strength while keeping the tablet dimensions and nominal weight the same means that the ratio of the drug substance to the other tablet excipients (filler, disintegrant, glidant, lubricant) changes. This results in different tablet characteristics and tablet performance. In order to obtain acceptable tablet characteristics, the composition needs to be reformulated. This means that for every dose to be provided, time consuming reformulation work is necessary, whether the tablet preparation is done batch-wise or by the above-mentioned continuous processes. Therefore, there is a need for tablets where the means of varying the dosage does not require reformulation of the composition or a change in the dimensions or nominal weight of the tablets, as well as a method to manufacture these tablets.

### SUMMARY OF THE INVENTION

The present invention relates to a multi-layered, physiologically tolerated oral dosage form for pharmaceutically active substances. The multi-layered dosage form comprises the following three layers: a central core, a middle layer and an outer shell, at least one of which includes at least one pharmaceutically active substance. By varying the diameter of the core, a different middle layer volume is obtained within a fixed outer shell dimension. Thus, the dosage strength can be adjusted by varying only the diameter of the core. This gives the ability to obtain different dosage strengths without the need of time-consuming reformulation work for the central core, middle layer, or outer shell. Moreover, the oral dosage form is produced in a simple, single step, continuous coating process where the core is coated with the middle layer and the outer shell. The central core can be produced by melt extrusion or solution spinning. The coating process can be done by extrusion or dipping. The materials for the core, the middle layer, and the outer shell can be selected in order to obtain the desired drug release characteristics. Both fast releasing dosage forms as well as slow releasing dosage forms can be obtained.

### BRIEF DESCRIPTION OF THE DRAWINGS

The novel features of the present invention will be more readily apparent from reading the following detailed description in conjunction with the drawings in which like elements in different figures are identified by the same reference numeral and wherein:
Fig. 1 is a schematic drawing of the multi-layer oral dosage form of the present invention, consisting of a central core, middle layer, and outer shell;
Fig. 2 is a cross-section of the multi-layer oral dosage form of the present invention shown in Fig. 1 taken along the 2---2 plane;
Fig. 3 is a perspective drawing of the central core section of an alternative embodiment of the present invention; and
Fig. 4 is a schematic drawing of a coating process for forming the multi-layer oral dosage form of the present invention.

### DETAILED DESCRIPTION OF THE INVENTION

Multi-layer oral dosage forms of the present invention are produced in the form of tablets, including oblong tablets, tablet shapes, capsule shapes and coated tablets, for oral applications.

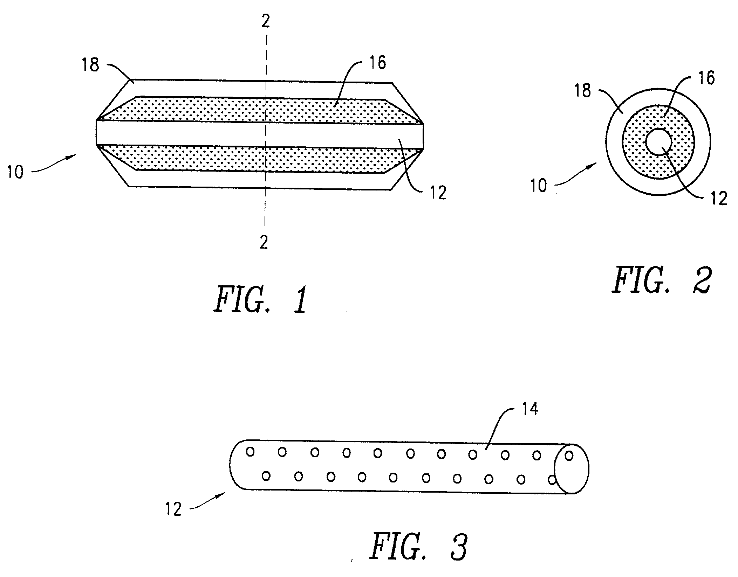

Referring to Figures 1 and 2, oral dosage form **10** prepared according to the invention consists of at least three layers: a core **12**, an overlay **16**, and a shell **18**. At least one of the layers contains at least one pharmaceutically active drug substance.

Preferably, the drug substance is included in overlay **16**, while core **12** will be inert. However, the drug substance may also be included in both core **12** and overlay **16.** This may allow for a fast release of drug substance from overlay **16** and a slow release of drug substance from core **12**.

Though schematically represented with circular cross-sections in Figure 2, one skilled in the art could envision the layers of oral dosage form **10** to be of other cross-sectional shapes such as elliptical or rounded rectangular. In addition, although this disclosure describes three layers, one could envision a structure containing a multitude of overlay **16** layers. Moreover, these many drug-containing overlay **16** layers may contain different drug substances in a variety of drug substance concentrations.

Overlay **16** is typically comprised of a drug substance and a carrier. The carrier of overlay **16** may consist of several components. These components include a thermoplastic, pharmacologically acceptable polymer or wax, or a blend of polymers and waxes. These polymers, waxes, or blends must be liquid or semi-liquid at room temperature or, alternatively, must melt or soften upon heating. The carrier can also consist of other components such as non-polymeric liquids. These include, but are not limited to oils, fats, or surfactants, and may also include excipients. It is important that melting or softening of the carrier occurs below the degradation temperature of any of the components or of the drug substances in overlay **16**.

Core **12** is comprised of a carrier as mentioned above, and may also contain a drug substance. The carrier of core **12** may consist of several components, including a thermoplastic, pharmacologically acceptable solid polymer or blend of polymers that melt or soften upon heating. The carrier can also contain other components such as excipients. For the production of core **12**, the polymer or polymer blend component must melt or soften below the degradation temperature of any of the other components or of any drug substances present in core **12**. During the process for applying overlay **16** and the shell **18** to core **12**, it is important that core **12** does not melt or soften in the range of the processing conditions of the process. Therefore, the polymer or polymer blend component of the carrier in core **12** must melt or soften in the range of 50°C to 350°C, preferably in the range of 150°C to 250°C.

Shell **18** is comprised of a carrier as mentioned above, and may also contain a drug substance. The carrier of shell **18** may consist of several components, including a thermoplastic, pharmacologically acceptable solid polymer or blend of polymers that melt or soften upon heating. The carrier can also contain other components such as excipients. Again the melting or softening temperatures must be below the degradation temperature of any of the components of shell **18**. Therefore, the polymer or polymer blend of shell **18** must melt or soften in the range of 50°C to 350°C, preferably in the range of 60°C to 250°C. Shell **18** may improve the surface finish of oral dosage form **10**, or may delay the drug release from overlay **16**.

The carrier for all of the layers of oral dosage form **10** can be crystalline, amorphous or a mixture of both amorphous and crystalline phases. Examples of suitable pharmacologically acceptable carriers for core **12**, overlay **16**, and shell **18** include, but are not limited to: cellulose ethers such as methylcellulose and ethylcellulose; hydroxyalkylcelluloses such as hydroxypropylcellulose and hydroxyalkyl alkylcelluloses such as hydroxyethyl methylcellulose and hydroxypropyl methylcellulose; carboxyalkylcelluloses such as carboxymethylcellulose, alkali metal salts of carboxyalkylcelluloses such as carboxymethylethylcellulose, carboxyalkylcellulose esters; cellulose phthalates such as cellulose acetate phthalate and hydroxypropylmethylcellulose phthalate; starches, thermoplastic starches, starch derivatives; sugar alcohols, such as mannitol; pectines such as sodium carboxymethylamylopectine; chitin derivatives such as chitosan; polysaccharides such as alginic acid, alkali metal and ammonium salts thereof carrageenans, galactomannans, tragacanth, agar-agar, gummi arabicum, guar gummi and xanthan gummi; polyhydroxyalkylacrylates; polyhydroxyalkylmethacrylates; polyacrylates; polymethacrylates (eudragit types); polyacrylic acids and salts thereof; polymethacrylic acids and salts thereof; methacrylate copolymers; polyvinylalcohol; polyvinylpyrrolidone, copolymers of polyvinylpyrrolidone and vinyl esters such as vinyl acetate; polyalkylene oxides such as polyethylene oxide and polypropylene oxide and copolymers of ethylene oxide and propylene oxide (poloxamer, pluronic); polyalcohols such as polyethylene glycol, polypropylene glycol; polyoxyethylene castor oils (cremophor); polyoxyethylene stearates; polyoxyethylene alkyl ethers; sesame oil; carnauba wax; mono- and diglycerides; triglycerides of the C12-, C14-, C16- and C18- fatty acids; polyalkylenes such as polyethylene and polypropylene; polyvinylidene; fluoropolymers such as polyvinylidenefluoride; polyurethanes; polyesters, polyamides, polylactic acid, polycaprolactone, polyglycolic acid, copolymers of polylactic acid and polycaprolactone, copolymers of polylactic acid and polyglycolic acid, copolymers of polycaprolactone, and polyglycolic acid, polydioxanone, copolymers of polydioxanone and polyglycolide, and copolymers of polydioxanone and polycaprolactone.

The preferred carrier for core **12** is poly(vinylidene fluoride).

The preferred carriers for overlay **16** are polyethylene glycols with a molecular weight between 200 Da and 20,000 Da.

The preferred carriers for shell **18** are hydroxyalkylcelluloses, polymethacrylates and copolymers of polyvinylpyrrolidone and vinyl esters such as vinyl acetate.

As previously mentioned, each of the layers as described herein above may further comprise one or more pharmaceutically acceptable excipients such as, for example, plasticizers, lubricants, flavors, colorants, stabilizers, complexing agents, surfactants, disintegrants and the like. Said ingredients should not be heat sensitive. That is, they should not show any appreciable degradation or decomposition within the range of temperatures to which the layers are exposed during the process to form oral dosage form **10**.

Plasticizers, for example, may be added to lower the glass transition of the polymer, which is advantageous where one of the components has limited thermal stability. Suitable pharmaceutically acceptable plasticizers include, but are not limited to low molecular weight polyalcohols such as ethylene glycol, propylene glycol, 1,2-butylene glycol, 2,3-butylene glycol, styrene glycol, polyethylene glycols such as diethylene glycol, triethylene glycol, tetraethylene glycol; polypropylene glycols; polyethylene-propyleneglycols; glycol ethers such as monopropylene glycol monoisopropyl ether, propylene glycol monoethyl ether, diethylene glycol monoethyl ether; ester type plasticizers such as aromatic carboxylic acid esters (e.g. dialkyl phtalates, trimellitic acid ester, benzoic acid esters, terephtalic acid esters), aliphatic dicarboxylic acid esters (e.g. citric acid esters, tartaric acid esters), monoethanolamine, diethanolamine, triethanolamine and the like. Of these, the low molecular weight polyethylene glycols are preferred. The concentration of the plasticizer is typically less than 30% by weight of the layer involved, preferably between 0.5% and 15% by weight of the layer involved.

Surfactants and complexing agents may be added to increase the solubility of the drug substance in any of the layers containing drug substances. For example suitable pharmaceutically acceptable surfactants are polyoxyethylene castor oils. Suitable complexing agents are cyclodextrines such as hydroxypropyl-beta-cyclodextrin.

At least one of the layers as described herein above contains at least one pharmaceutically active drug substance. Preferably, the drug substance is located in overlay **16**. In principal, any pharmaceutically active drug substance that does not decompose under the processing conditions can be used with the present invention. Suitable active ingredients are those which exert a local physiological effect, as well as those which exert a systemic effect, after oral administration. Examples thereof are:
- analgesic and anti-inflammatory drugs (NSAIDs, fentanyl, indomethacin, ibuprofen, ketoprofen, nabumetone, paracetamol, piroxicam, tramadol, COX-2 inhibitors such as celecoxib and rofecoxib) ;
- anti-arrhythmic drugs (procainamide, quinidine, verapamil);
- antibacterial and antiprotozoal agents (amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, norfloxacin, ofloxacin, oxacillin, phenoxymethyl-penicillin potassium, pyrimethamine-sulfadoxime, streptomycin);
- anti-coagulants (warfarin, reparin);
- antidepressants (amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline; 3-[2-[3,4-dihydrobenzofuro[3,2-c]pyridin-2(1*H*)-yl]ethyl]-2-methyl-4*H*-pyrido[1,2-a]pyrimidin-4-one);
- anti-diabetic drugs (glibenclamide, metformin);
- anti-epileptic drugs (carbamazepine, clonazepam, ethosuximide, gabapentin, lamotrigine, levetiracetam, phenobarbitone, phenytoin, primidone, tiagabine, 2,3:4,5-bis-O-(1-methylethylidene)-β-D-fructopyranose sulfamate, valpromide, vigabatrin);
- antifungal agents (amphotericin, clotrimazole, econazole, fluconazole, flucytosine, griseofulvin, itraconazole, ketoconazole, miconazole nitrate, nystatin, terbinafine, voriconazole);
- antihistamines (astemizole, cinnarizine, cyproheptadine, decarboethoxyloratadine, fexofenadine, flunarizine, levocabastine, loratadine, norastemizole, oxatomide, promethazine, terfenadine);
- anti-hypertensive drugs (captopril, enalapril, ketanserin, lisinopril, minoxidil, prazosin, ramipril, reserpine, terazosin);
- anti-muscarinic agents (atropine sulphate, hyoscine);
- antineoplastic agents and antimetabolites (platinum compounds, such as cisplatin, carboplatin; taxanes, such as paclitaxel, docetaxel; tecans, such as camptothecin, irinotecan, topotecan; vinca alkaloids, such as vinblastine, vindecine, vincristine, vinorelbine; nucleoside derivatives and folic acid antagonists such as 5-fluorouracil, capecitabine, gemcitabine, mercaptopurine, thioguanine, cladribine, methotrexate; alkylating agents, such as the nitrogen mustards, e.g. cyclophosphamide, chlorambucil, chlormethine, iphosphamide, melphalan, or the nitrosoureas, e.g. carmustine, lomustine, or other alkylating agents, e.g. busulphan, dacarbazine, procarbazine, thiotepa; antibiotics, such as daunorubicin, doxorubicin, idarubicin, epirubicin, bleomycin, dactinomycin, mitomycin; HER 2antibody, such as trastuzumab; podophyllotoxin derivatives, such as etoposide, teniposide; farnesyl transferase inhibitors; anthrachinon derivatives, such as mitoxantron);
- anti-migraine drugs (alniditan, naratriptan, sumatriptan);
- anti-Parkinsonian drugs (bromocryptine mesylate, levodopa, selegiline);
- antipsychotic, hypnotic and sedating agents (alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone, zolpidem);
- anti-stroke agents (lubeluzole, lubeluzole oxide, riluzole, aptiganel, eliprodil, remacemide);
- antitussive (dextromethorphan, laevodropropizine);
- antivirals (acyclovir, ganciclovir, loviride, tivirapine, zidovudine, lamivudine, zidovudine + lamivudine, zidovudine+lamivudine+abacavir, didanosine, zalcitabine, stavudine, abacavir, lopinavir, lopinavir+ritonavir, amprenavir, nevirapine, efavirenz, delavirdine, indinavir, nelfinavir, ritonavir, saquinavir, adefovir, hydroxyurea);
- beta-adrenoceptor blocking agents (atenolol, carvedilol, metoprolol, nebivolol, propanolol);
- cardiac inotropic agents (amrinone, digitoxin, digoxin, milrinone;
- corticosteroids (beclomethasone dipropionate, betamethasone, budesonide, dexamethasone, hydrocortisone, methylprednisolone, prednisolone, prednisone, triamcinolone);
- disinfectants (chlorhexidine);
- diuretics (acetazolamide, frusemide, hydrochlorothiazide, isosorbide);
- enzymes;
- essential oils (anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol, thyme);
- gastro-intestinal agents (cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel, sulphasalazine);
- immunosurpressive agents (rapamycin);
- haemostatics (aminocaproic acid, thrombin);
- lipid regulating agents (atorvastatin, lovastatin, pravastatin, probucol, simvastatin);
- local anaesthetics (benzocaine, lidocaine, bupivaocaine);
- opioid analgesics (buprenorphine, codeine, dextromoramide, dihydrocodeine, hydrocodone, oxycodone, morphine);
- parasympathomimetics and anti-dementia drugs (AIT-082, eptastigmine, galanthamine, metrifonate, milameline, neostigmine, physostigmine, tacrine, donepezil, rivastigmine, sabcomeline, talsaclidine, xanomeline, memantine, lazabemide);
- peptides and proteins (antibodies, becaplermin, cyclosporine, erythropoietin, immunoglobulins, insuline);
- sex hormones (oestrogens : conjugated oestrogens, ethinyloestradiol, mestranol, oestradiol, oestriol, oestrone; progestogens ; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone, quingestanol acetate);
- stimulating agents (sildenafil);
- vasodilators (amlodipine, buflomedil, amyl nitrite, diltiazem, dipyridamole, glyceryl trinitrate, isosorbide dinitrate, lidoflazine, molsidomine, nicardipine, nifedipine, oxpentifylline, pentaerythritol tetranitrate);
their *N*-oxides, their pharmaceutically acceptable acid or base addition salts and their stereochemically isomeric forms.

The pharmaceutically active drug substances can be suspended or dissolved in the carrier of overlay **16**. If the carrier is a solid polymer or wax as described herein above, the term solid dispersion is used. A solid dispersion defines a system in a solid state comprising at least two components, wherein one component is dispersed more or less evenly throughout the other component or components.. When said solid dispersion is such that the system is chemically and physically uniform or homogeneous throughout or consists of one phase at the molecular level, such a solid dispersion will be called a solid solution. Solid solutions are preferred physical systems for poorly water soluble drugs because the components therein show a higher aqueous solubility and eventually a higher bio-availability to the organisms to which they are administered. The term solid dispersion also comprises dispersions which are less homogeneous throughout than solid solutions. Such dispersions are not chemically and physically uniform throughout or they may comprise more than one phase. For example, the term solid dispersion also relates to other combinations, including, but not limited to, two or more amorphous phases, an amorphous phase with a crystalline phase, or two or more crystalline phases.

The release of the drug substance can be modified by the proper selection of the materials for each layer. This is clear to someone who is skilled in the art and it should be understood that the different possibilities are not limited to these listed below.

For example, a fast release can be obtained by an overlay **16** and shell **18** that dissolve rapidly into aqueous media. Preferred materials for overlay **16** to obtain a fast release are polyethylene glycols with molecular weight in the range of 200 Da to 20,000 Da, such as PEG 200 and PEG 10,000 (sold by Aldrich Chemicals, Milwaukee, WI). Preferred materials for shell **18** to obtain a fast release are polymethacrylates (pH < 5), such as that sold under the tradename EUDRAGIT E100 by Rohm GmbH of Darmstadt, Germany, and copolymers of polyvinylpyrrolidone and vinyl esters, such as that sold under the tradename KOLLIDON VA 64 by BASF, Ludwigshafen, Germany.

A slow release can be obtained by, for example, a slowly dissolving shell **18**. Preferred materials for shell **18** to obtain a slow release are hydroxyalkylcelluloses such as HPC 150-700 cps, sold under the tradename KLUCEL EF by Hercules Incorporated, Aqualon Division, Wilmington, DE.

It is also possible to disperse the drug substance in both the overlay **16** and core **12**. This allows for a fast releasing component from overlay **16** (with fast dissolving shell **18**) and a slow releasing component from core **12**. For this purpose, preferred materials for overlay **16** are polyethylene glycols with molecular weight in the range of 200 Da to 20,000 Da and for core **12** poly(vynilidene fluoride), or PVDF, is preferred.

It is also possible to obtain a slow releasing oral dosage form **10** by an alternative embodiment of the present invention. In this case, a water insoluble shell **18** and core **12** are used. Figure 3 shows a perspective view of core **12** that may be used in this embodiment. As shown in the figure, core **12** is hollow and perforated throughout the length by pores **14**. Core **12** further foresees open ends at one or both sides of dosage form **10**. This allows gastric or intestinal fluids to enter dosage form **10** through perforated core **12**. Drug is released by diffusion through pores **14** and release rate is determined by the size and number of pores **14**. Pores **14** can be obtained for example using a laser beam. Suitable pharmaceutical acceptable polymers for the water insoluble shell **18** and core **12** include polyalkylenes such as polyethylene and polypropylene, polyurethanes, and fluoropolymers.

The multi-layer oral dosage form **10** as described herein above may be produced by a coating process whereby core **12** is coated with overlay **16** and shell **18**. The coating can be performed by extrusion or dipping.

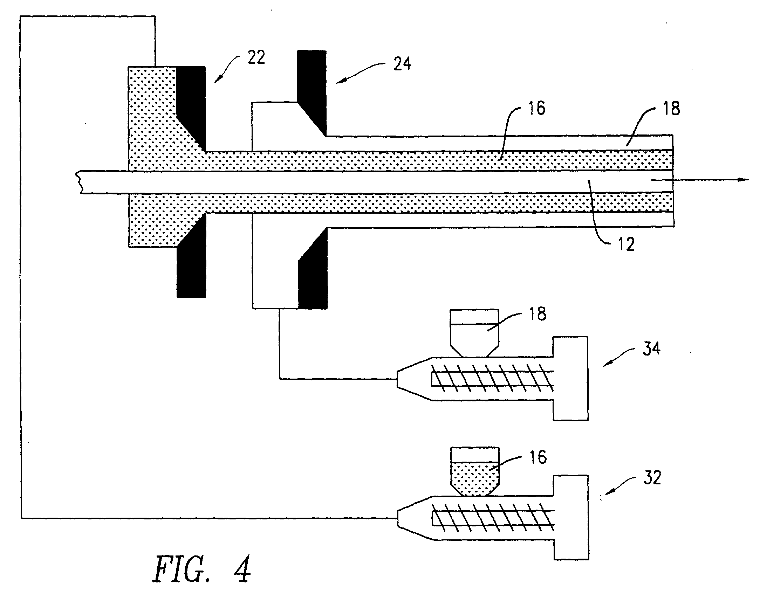

Preferably, the coating is done by extrusion, whereby a coating die is used to combine core **12**, overlay **16**, and shell **18**. Figure 4 shows a schematic presentation of the coating process. More particularly, Figure 4 shows core **12** moving through a set of dies 22, 24 as follows. Core **12** first passes through overlay coating die **22** where overlay **16** is deposited on core **12**. The core **12**/overlay **16** combination then passes through shell coating die **24** where shell **18** is deposited on overlay **16**. The materials for overlay **16** and shell **18** are supplied to the regions of overlay coating die **22** and shell coating die **24** by extruders **32** and **34**, respectively. It must be noted that overlay coating die **22** and shell coating die **24** could be constructed so that shell **18** and overlay **16** are deposited on core **12** simultaneously.

Coating dies **22,24** are typically annular nozzles with openings, allowing the combining of different streams into one strand. The diameter of the die openings, together with the temperature and throughput, determines the final diameter of the layers in oral dosage form **10.**

Core **12** may be produced by melt extrusion or solution spinning. Preferably, core **12** is produced by a melt extrusion process. This is advantageous since melt extrusion is a solvent free process. Melt extrusion is performed using a melt extruder and may use the following steps:
- feed the components (gravimetric feeders) or a pre-mix to the extruder or melt container with a metering pump and heat the blend until a homogeneous melt is obtained,
- pump the melt through a die, and
- cool the melt until it solidifies.

The term melt or melting should be interpreted broadly. For our purposes, these terms not only mean the alteration from a solid state to a liquid state, but can also refer to a transition from a glassy state to a rubbery state or even a softening of the materials. The size or diameter of the die opening will determine the final diameter of core **12**. For a circular cross-section, the diameter of core **12** is preferably between 0.1 and 10 mm, most preferably between 0.5 and 6 mm. For the purpose of the coating process, the solidified core **12** is further guided to the coating process and pulled through the inner openings of dies **22** and **24**.

Before overlay **16** is deposited on core **12**, the pharmaceutically active substance, carrier and optional additives need to be mixed in order to obtain a homogenous mixture. This can be done in extruder **32** by feeding the components (e.g., by gravimetric feeder) or pre-blend into extruder **32**, mixing the components until one obtains a homogenous melt, and supplying the mixture of components for overlay **16** to the region of overlay coating die **22**.

The inner diameter of overlay coating die **22** determines the outer diameter of drug containing overlay **16**. For a circular cross-section, the outer diameter of drug containing overlay **16** is preferably between 0.1 and 10 mm, most preferably between 3 and 8 mm. Since the diameter is fixed for a given overlay coating die **22**, the outer diameter of overlay **16** is also fixed for a given set of process conditions. One is now able to pull different diameters of core **12** through overlay coating die **22**, resulting in different overlay **16** volumes, which in turn results in different dosage strengths for the same components of overlay **16**.

Before shell **18** is introduced in shell coating die **24**, the polymer and optionally additives need to be mixed in order to obtain a homogenous mixture. This can be done in extruder **34** by feeding the components (gravimetric feeder) or pre-blend into extruder **34**, mixing and/or heating the components until one obtains a homogenous melt, and supplying the mixture of components for shell **18** to the region of shell coating die **24**.

The inner diameter of shell coating die **24** determines the outer diameter of shell **18**. It must be noted that the dimensions of multi-layer oral dosage form **10** must be small enough to allow for a human or other mammal to swallow. For a circular cross-section, the outer diameter of shell **18** is preferably between 0.1 and 10 mm, most preferably between 3 and 8 mm.

After being forced or pumped through coating dies **22**, **24**, the multi-layered strand is cooled on a cooling conveyer. Cooling can be done using an air-knife or a cooling liquid which circulates through the conveyer. In some cases quenching may be necessary, in other cases natural air cooling is sufficient.

The still deformable multi-layered strand can then be shaped and cut online into the desired oral dosage form **10**. Preferably, the dosage form **10** is tablet or capsule like shaped. This can be done in a number of different ways as described in the art.

The following non-limiting examples demonstrate the invention. To test the feasibility of the core materials, concept placebo tablets and drug-containing tablets were prepared composed of different core, overlay and shells. The forming of concept placebo tablets is described in Examples 1 and 2. The forming of, and drug release from, drug-containing tablets are described in Example 3.

### Example 1:

Concept placebo tablets prepared in this example consisted of a core, an overlay, and a shell. The core was composed of a physical blend of Klucel EF (HPC 150-700 cps, Aqualon, Zwijndrecht, The Netherlands) and methylparaben, or methyl 4-hydroxybenzoate, (Aldrich Chemicals, Milwaukee, WI) in a 90/10 w/w ratio. The overlay was PEG 200 (Aldrich Chemicals, Milwaukee, WI) and the shell was Eudragit E100, (Rohm Pharma, Darmstadt, Germany).

The core was prepared using a single screw extruder (Plasticorder, C.W. Brabender, Hackensack, NJ). The screw had a diameter of 0.75-inch, an L/D ratio of 25:1 and a constant compression ratio of 2.5:1. A coating die (B & H Tool Co. Inc., San Marcos, CA) was installed at the outlet of the extruder with a closed tip with an outer diameter of 1.4 mm. The barrel was electrically heated at three different heating zones (T₁ = 20, T₂ = 160, T₃= 180^{º}C) and at the die (T_{die} = 180^{º}C). The feeding zone was cooled with water. The other variable parameter was the screw speed (V = 20 rpm). Based on the settings of these parameters, a value of 14 Pa for the torque (P) was obtained.

After extrusion, the core was cooled on a conveyer (C.W. Brabender, Hackensack, NJ) with an air knife (Exair, Cincinnati, OH), and taken up by a roller-puller (Harrel, IL). The final diameter of the core was determined by the diameter of the coating die (1.4 mm) and the take up speed of the roller-puller (14 feet per minute). After leaving the roller-puller, the core was wound on a spool (Progressive Machine Company, Ringwood, NJ).

The overlay and shell were simultaneously coated on the core. The above mentioned extruder was used. At the outlet of the extruder, a coating die (B & H Tool Co. Inc., San Marcos, CA) was installed with an outer ring diameter of 5 mm and an open tip with an inner diameter of 2.4 mm. The core was fed through the 2.4 mm diameter open tip of the coating die. The PEG 200 overlay was also fed into the open tip of the coating die using a pump (Model nº1, Zenith, Sanford, NC) at 7.5 rpm (0.584 cc/revolution). The molten shell was pumped into the outer ring of the die by the extruder. The barrel of the extruder was electrically heated at three different heating zones (T₁ = 120, T₂ = 135, T₃= 135^{º}C) and at the die (T_{die} = 135^{º}C). The feeding zone was cooled with water. The screw speed (V) was 20 rpm), which resulted in a value of 80-85 Pa for the torque (P).

Upon exiting the coating die, the multi-layered strand was cooled on the above described cooling conveyer with the air knife. The still deformable multi-layered strand was then formed into tablets with an embedded cutting roll. The cutting went well and tablets were sealed at both side ends.

### Example 2:

Concept placebo tablets prepared in this example consisted of a core, an overlay, and a shell. The overlay and the shell were the same as Example 1. The core was a 0.5 mm diameter strand of polyvinylidenefluoride (PVDF) from Ethicon Incorporated, Somerville, NJ, (diameter 0.5 mm).

Like Example 1, the overlay and shell were simultaneously coated on the core. The core was fed through the 2.4 mm diameter open tip of the coating die. The PEG 200 overlay was also fed into the open tip of the coating die using an overpressure of 0.2 Pa from the pressurized vessel. The molten shell was pumped into the outer ring of the die by the extruder. The barrel of the extruder was electrically heated at three different heating zones (T₁ = 125, T₂ = 135, T₃= 135°C) and at the die (T_{die} = 135°C) . The feeding zone was cooled with water. The screw speed (V) was 20 rpm, which resulted in a value of 65 Pa for the torque (P).

Upon exiting the coating die, the multi-layered strand was cooled on the cooling conveyer with the air knife described in Example 1. The still deformable multi-layered strand was then formed into tablets with an embedded cutting roll. The cutting went well and tablets were sealed at both side ends.

### Example 3:

The forming of, and drug release from, 2,3:4,5-bis-O-(1-methylethylidene)-β-D-fructopyranose sulfamate containing tablets are described in this example. Tablets prepared in this example consisted of a core, an overlay, and a shell. The core was the same 0.5 mm diameter strand of PVDF as described in Example 2. The overlay was a blend of PEG 200, PEG 10000 (Aldrich Chemicals, Milwaukee, WI), and D-2,3:4,5-bis-O-(1-methylethylidene)-β-B-fructopyranose sulfamate. The shell was Eudragit E100.

The overlay was prepared as follows: 50 gms of PEG 10000 was melted in a glass beaker on a hot plate at 100^{º}C. Then 50 gms of 2,3:4,5-bis-O-(1-methylethylidene)-β-D-fructopyranose sulfamate was added while stirring with a magnetic bar. After the drug was dissolved in the molten PEG 10000, 200 gms of PEG 200 was added while mixing with a magnetic bar until a clear solution was obtained. The overlay was then transferred to the melt container.

Like Example 1, the overlay and shell were simultaneously coated on the core. The core was fed through the 2.4 mm diameter open tip of the coating die. To change the thickness of the overlay, two trials were performed. In the first, one 0.5 mm diameter strand of PVDF was fed through the coating die. In the second, three 0.5 mm diameter strands of PVDF were fed through the coating die.

The overlay was also fed into the open tip of the coating die at 100^{º}C using the Zenith Model n^{º}1 pump of Example 1, at 15 rpm (0.584 cc/revolution). The molten shell was pumped into the outer ring of the die by the extruder. The barrel of the extruder was electrically heated at three different heating zones (T₁ = 120, T₂ = 135, T₃= 135^{º}C) and at the die (T_{die} = 135^{º}C). The feeding zone was cooled with water. The screw speed (V) was 20 rpm, which resulted in a value of 55-65 Pa for the torque (P).

Upon exiting the coating die, the multi-layered strand was cooled on the cooling conveyer with the air knife described in Example 1. The still deformable multi-layered strand was then formed into tablets with an embedded cutting roll. The cutting went well and tablets were sealed at both side ends.

The dimensions of the tablets from both trials were similar, as both trials yielded tablets approximately 3 mm thick. To determine the dose of 2,3:4,5-bis-O-(1-methylethylidene)-β-D-fructopyranose sulfamate in each tablet, the tablet was dissolved in 10 ml H₂O/0.1 N HCl 9/1 v/v. The concentration of 2,3:4,5-bis-O-(1-methylethylidene)-β-D-fructopyranose sulfamate was analyzed by HPLC (Waters System with Millenium Software, 2690 Alliance, Waters, Milford, MA). A sample of 50 uL is injected into a Zorbax Eclipse (HP, Palo Alto, CA) column (XDB-C8, 4.6*150 mm, P/N: 993967.906). The mobile phase consists of H₂O/methanol 68/32 w/w at a flow rate of 1.5 mL/min. The concentration was determined with a refractive index detector (Sensitivity 32). The peak retention time is 6.7 minutes and the run takes 14 minutes.

The dose analysis showed that tablets made in Trial 1 (one strand of PVDF as core) averaged 13.9 mg of 2,3:4,5-bis-O-(1-methylethylidene)-β-D-fructopyranose sulfamate, while the tablets made in Trial 2 (three strands of PVDF as core) averaged 10.7 mg of 2,3:4,5-bis-O-(1-methylethylidene)-β-D-fructopyranose sulfamate. These results show that tablets with a comparable thickness but a different dose are obtained when a different core diameter is used.

The *in vitro* release of the tablets made in this example was also determined. Four tablets from Trial 2 were placed in a USP II apparatus (SR8 plus, Hanson, Chatsworth, CA) containing 250 ml of 0.1 N HCl at 37°C and a paddle rotating at 50 rpm. Dissolution was followed up to 1 hour, with samples taken after 5, 15, 30 and 60 minutes. An aliquot of 5 ml was filtered through a PTFE 0.2 micron filter. The sample was not replaced with fresh solvent. The concentration of 2,3:4,5-bis-O-(1-methylethylidene)-β-D-fructopyranose sulfamate was analyzed by HPLC as discussed above.

The dissolution study showed that after 5 minutes, approximately 4 percent of the 2,3:4,5-bis-O-(1-methylethylidene)-β-D-fructopyranose sulfamate had been released. By 15 minutes, about 48 percent had been released, and complete release was obtained in 30 minutes.

Accordingly, there has been disclosed a multi-layered oral dosage form for pharmaceutically active substances and a method for producing same. While illustrative embodiments have been disclosed, it is understood that variations to the disclosed embodiments are possible, and it is intended that this invention be limited only by the scope of the appended claims.

## Claims

1. A multi-layered oral dosage form for pharmaceutically active substances, comprising:
a central core having a volume;
a middle layer surrounding said central core; and
an outer shell,
at least one of said central core, said middle layer and said outer shell including at least one pharmaceutically active substance,
wherein the volume of said central core is selected to obtain a desired volume of said middle layer within a predetermined dimension of said outer shell.

2. The form according to Claim 1, wherein said central core is a solid cylinder, a hollow cylinder or a perforated hollow cylinder.

3. The form according to Claim 1 or Claim 2, wherein said central core, said middle layer or said outer shell includes said at least one pharmaceutically active substance.

4. The form according to any one of Claims 1 to 3, wherein:
said central core includes at least one of a thermoplastic pharmacologically acceptable solid polymer that melts or softens upon heating, a blend of thermoplastic pharmacologically acceptable polymers that melt or soften upon heating or an excipient as a carrier;
said outer shell includes at least one of a thermoplastic pharmacologically acceptable solid polymer that melts or softens upon heating, a blend of thermoplastic pharmacologically acceptable polymers that melt or soften upon heating or an excipient as a carrier; and
said middle layer includes at least one of a thermoplastic pharmacologically acceptable polymer that melts or softens upon heating, a blend of thermoplastic pharmacologically acceptable polymers that melts or softens upon heating, a thermoplastic pharmacologically acceptable wax that melts or softens upon heating, a blend of thermoplastic pharmacologically acceptable waxes that melts or softens upon heating, a blend of said polymers and said waxes, a non-polymeric liquid or an excipient as a carrier.

5. The form according to Claim 4, wherein said thermoplastic pharmacologically acceptable solid polymer or polymers are at least one of a cellulose ether, a hydroxyalkylcellulose, a carboxyalkylcellulose, an alkali metal salt of a carboxyalkylcellulose, a cellulose phthalate, a starch, a thermoplastic starch, a starch derivative, a sugar alcohol, a pectine, a chitin derivative, a polysaccharide or an alkali metal or ammonium salt thereof, a carrageenan, a galactomannan, tragacanth, agar-agar, gummi arabicum, guar gummi, xanthan gummi, a polyhydroxyalkylacrylate, a polyhydroxyalkylmethacrylate, a polyacrylate, a polymethacrylate (eudragit types), a polyacrylic acid or a salt thereof, a polymethacrylic acid or a salt thereof, a methacrylate copolymer, polyvinylalcohol, polyvinylpyrrolidone, a copolymer of polyvinylpyrrolidone, a vinyl ester, a polyalkylene oxide or a copolymer of ethylene oxide and propylene oxide, a polyalcohol, a polyoxyethylene castor oil, a polyoxyethylene stearate, a polyoxyethylene alkyl ether, sesame oil, camauba wax, a mono- or di-glyceride, a triglyceride of a C12-, C14-, C16- or C18- fatty acid, a polyalkylene, a polyvinylidene, a fluoropolymer, a polyurethane, a polyester, a polyamide, a polylactic acid, a polycaprolactone, a polyglycolic acid, a copolymer of polylactic acid and polycaprolactone, a copolymer of polylactic acid and polyglycolic acid, a copolymer of polycaprolactone, and polyglycolic acid, polydioxanone, a copolymer of polydioxanone and polyglycolide or a copolymer of polydioxanone and polycaprolactone.

6. The form according to Claim 4 or Claim 5, wherein said carrier included in said central core is poly (vinylidene fluoride).

7. The form according to any one of Claims 4 to 6, wherein said carrier included in said outer shell is hydroxyalkylcellulose, polymethacrylate, a copolymer of polyvinylpyrrolidone or a vinyl ester.

8. The form according to any one of Claims 4 to 7, wherein said carrier included in said middle layer is a polyethylene glycol with a molecular weight in the range from 200 Da to 20,000 Da.

9. The form according to any one of Claims 1 to 8, wherein said at least one pharmaceutically active substance is an analgesic or anti-inflammatory drug, an anti-arrhythmic drug, an antibacterial or antiprotozoal agent, an anti-coagulant, an antidepressant, an anti-diabetic drug, an anti-epileptic drug, an antifungal agent, an antihistamine, an anti-hypertensive drug, an anti-muscarinic agent, an antineoplastic agent an antimetabolite, an anti-migraine drug, an anti-Parkinsonian drug, an antipsychotic, a hypnotic, a sedating agent, an anti-stroke agent, an antitussive agent, an antiviral, a beta-adrenoceptor blocking agent, a cardiac inotropic agent, a corticosteroid, a diuretic, an enzyme, an essential oil, a gastro-intestinal agent, an immunosurpressive agent, a haemostatic, a lipid regulating agent, a local anaesthetic, an opioid analgesic, a parasympathomimetic, a anti-dementia drug, a peptide, a protein, a sex hormone, a stimulating agent or a vasodilator.

10. A method for producing a multi-layered oral dosage form for pharmaceutically active substances, wherein the outer dimension of said form is fixed and the volume of a middle layer is selectively variable, comprising the steps of:
providing a core having a selected volume;
coating said core with said middle layer, wherein the total combined volume of said core and said middle layer is a predetermined volume; and
coating said middle layer with an outer shell to obtain said fixed outer dimension, wherein at least one of said core, said middle layer and said outer shell includes at least one pharmaceutically active substance.
